# EUROPÄISCHE PATENTSCHRIFT

(11) **EP 0 860 699 B1**
(45) Veröffentlichungstag und Bekanntmachung des Hinweises auf die Patenterteilung: **24.10.2001**
(21) Anmeldenummer: 98102822.8
(22) Anmeldetag: 18.02.1998
(51) Int. Cl.: G01N 33/28

(54) **Anordnung zum Überwachen von mehreren, mit Öl gefüllten elektrischen Einrichtungen**
System for monitoring several oil filled electrical devices
Système de surveillance de plusieurs dispositifs électriques remplis de l'huile

(30) Priorität: 19.02.1997 DE 19706530
(43) Veröffentlichungstag der Anmeldung: 26.08.1998
(73) Patentinhaber: SIEMENS AKTIENGESELLSCHAFT, 80333 München (DE)
(72) Erfinder: Müller, Friedhelm, 76351 Linkenheim-Hochstetten (DE)

(56) Entgegenhaltungen:
- DE-A- 3 227 631
- DE-A- 4 339 536
- US-A- 3 827 302
- US-A- 4 236 404
- US-A- 4 402 211
- US-A- 5 659 126
- PATENT ABSTRACTS OF JAPAN vol. 009, no. 251 (E-348), 8. Oktober 1985 (1985-10-08) & JP 60 102710 A (HITACHI SEISAKUSHO KK), 6. Juni 1985 (1985-06-06)

## Beschreibung

Die Erfindung betrifft eine Anordnung nach dem Oberbegriff des Anspruchs 1.

Eine derartige, aus der US-A-4 402 211 oder der DE-A-3 227 631 bekannte Anordnung dient zum Überwachen ölgefüllter elektrischer Einrichtungen, wie z. B. Transformatoren oder Drosseln. Dazu werden die in dem Öl gelösten Gase analysiert und so Betriebsstörungen der elektrischen Einrichtung über Änderungen der Gasanteile infolge von Überhitzung oder elektrischen Entladungen detektiert. Zur Extraktion der in dem Öl gelösten Gase dient ein durch eine semipermeable Membran gegenüber dem Öl abgetrenntes Gasaufnahmevolumen, in dem die eindiffundierenden Gase angereichert werden. Das Gasaufnahmevolumen ist über ein Umschaltventil an einer Leitung angeschlossen, die an einem Ende mit einer Trägergasquelle und an dem anderen Ende mit einem als Gasanalyseeinrichtung dienenden Gaschromatographen verbunden ist. Je nach Schaltstellung des Umschaltventils wird das Trägergas durch das Gasaufnahmevolumen hindurch oder an diesem vorbeigeleitet, wobei im ersten Fall die angereicherten Gase von dem Trägergas aus dem Gasaufnahmevolumen heraus in die Gasanalyseeinrichtung geschoben werden und im zweiten Fall die Gasanalyseeinrichtung durch das Trägergas gereinigt wird.

Weitere derartige Anordnungen sind aus der US-A-4 058 373 und der US-A-4 112 737 bekannt.

Zur Off-line-Überwachung mehrerer ölgefüllter elektrischer Einrichtungen ist entsprechend der US-A-4 402 411 jeder dieser Einrichtungen jeweils ein Gasaufnahmevolumen mit Umschaltventil zugeordnet, wobei an dem Umschaltventil Anschlüsse zum lösbaren Anschließen des Umschaltventils an die Leitung mit der Trägergasquelle und der Gasanalyseeinrichtung vorgesehen sind. Die Trägergasquelle, die Gasanalyseeinrichtung und die sie verbindende Leitung sind zusammen in einer tragbaren Einheit angeordnet, die an jeder einzelnen zu überwachenden elektrischen Einrichtung anschließbar ist.

Aus der US-A-3 827 302 ist es bekannt, mehrere der oben erwähnten Umschaltventile an der ihnen gemeinsamen Leitung anzuschließen und die Umschaltventile zyklisch umzuschalten, um der Gasanalyseeinrichtung unterschiedliche Proben nacheinander zuführen zu können. Dabei ist jedem Umschaltventil ein dem oben erwähnten Aufnahmevolumen entsprechendes Dosiervolumen für die jeweilige Probe zugeordnet.

Aus der DE-A-43 39 536 ist es bekannt, eine Probe mittels eines Trägergases einer gaschromatographischen Analyseeinrichtung mit Trennsäule und nachgeordnetem Detektor über ein Dosierventil mit Dosiervolumen derart zuzuführen, dass die Probe die Trennsäule mindestens zweimal durchläuft, bevor sie zu dem Detektor gelangt.

Der Erfindung liegt die Aufgabe zugrunde, mit möglichst geringem gerätetechnischen Aufwand eine störungsfreie On-line-Überwachung ölgefüllter elektrischer Einrichtungen zu ermöglichen.

Gemäß der Erfindung wird die Aufgabe durch die in Anspruch 1 angegebene Anordnung gelöst.

Eine vorteilhafte Weiterbildung der erfindungsgemäßen Anordnung ist dem Unteranspruch zu entnehmen.

Die erfindungsgemäße Anordnung ermöglicht in vorteilhafter Weise eine On-line-Überwachung aller ölgefüllten elektrischen Einrichtungen, z. B. den Transformatoren und Drosseln einer Umspannstation, mit nur einer Gasanalyseeinrichtung, indem durch zyklisches Umschalten der Umschaltventile das Trägergas nacheinander durch jedes einzelne Gasaufnahmevolumen geleitet wird und die darin befindlichen angereicherten Gase zu der Gasanalyseeinrichtung fördert. Dabei werden immer nur so viele Gasaufnahmevolumina gleichzeitig von dem Trägergas durchströmt, wie es der räumliche Abstand, also die Länge der Leitungsabschnitte zwischen den einzelnen Gasaufnahmevolumina, zuläßt, ohne daß es dabei zu einer Vermischung der Gase aus unterschiedlichen Gasaufnahmevolumina kommt. Im einfachsten und sichersten Fall wird daher immer nur eines der Gasaufnahmevolumina von dem Trägergas durchströmt. Die zur Umschaltung der Umschaltventile dienende Steuereinrichtung stellt eine entsprechende Umschaltinformation bereit, so daß eine exakte Zuordnung der einzelnen Ergebnisse der Gasanalyse zu den jeweiligen ölgefüllten elektrischen Einrichtungen, von denen die analysierten Gase kommen, gegeben ist.

Das vorgesehene steuerbare Dosierventil ermöglicht es, der Gasanalyseeinrichtung für jede einzelne Gasanalyse eine durch ein Dosiervolumen definierte Menge der zu analysierenden Gase zuzuführen und zwischen den einzelnen Gasanalysen die Gasanalyseeinrichtung mit dem Trägergas zu spülen. Wenn die in den Gasaufnahmevolumina angereicherten Gase von dem Trägergas wie ein Pfropf durch die Leitung geschoben werden, kommt es in den beiden Endbereichen des Pfropfes zu störenden Vermischungen der zu analysierenden Gase mit dem Trägergas; das vorgesehene Dosiervolumen ermöglicht in vorteilhafter Weise eine Gasentnahme aus dem mittleren Bereich des Pfropfes ohne die störenden Endbereiche.

Der vorgesehene Gasdetektor detektiert die Ankunft der Pfropfe mit den zu analysierenden Gasen im Bereich des Dosiervolumens und steuert das Dosierventil dementsprechend zur exakten Entnahme der definierten Gasmenge.

Der sich beim Ausspülen der Gasaufnahmevolumina in diesen einstellende Druck ist u. a. von der Position der Gasaufnahmevolumina in der sie verbindenden Leitung abhängig und somit nicht überall gleich, was die Gasanreicherung in den einzelnen Gasaufnahmevolumina unterschiedlich beeinflußt. Durch die im Unteranspruch angegebene Weiterbildung der erfindungsgemäßen Anordnung wird ein Druckausgleich ermöglicht, wozu alle Gasaufnahmevolumina nach dem Ausspülen mit dem Trägergas durch kurzzeitiges Öffnen der Ventile mit der Atmosphäre verbunden werden.

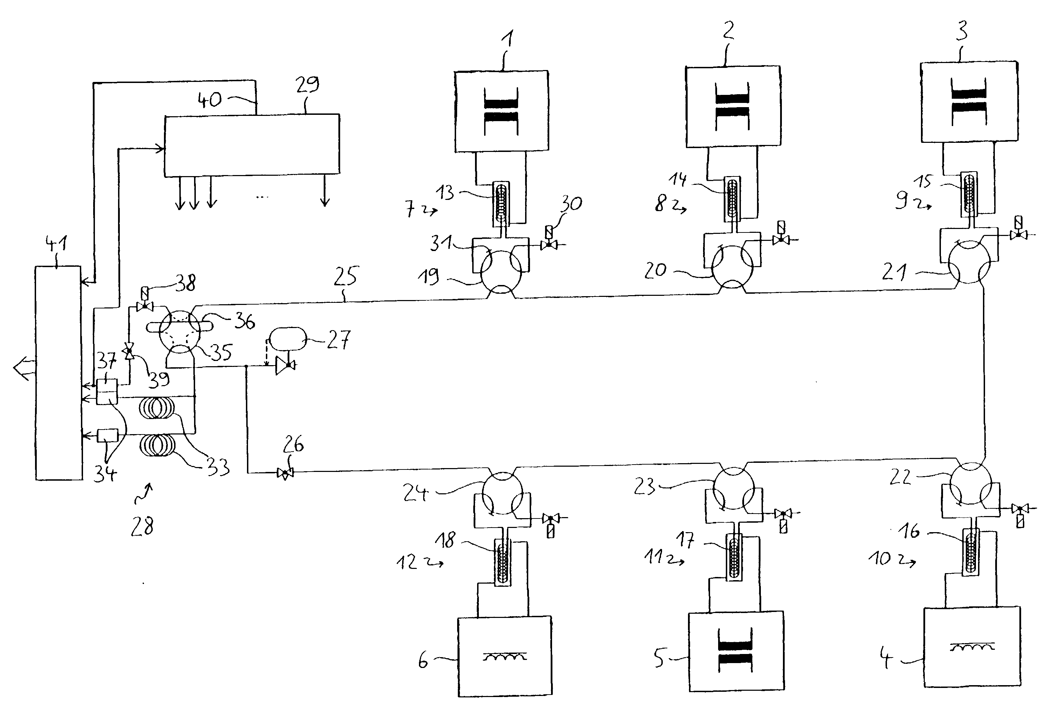

Zur weiteren Erläuterung der Erfindung wird im folgenden auf die Figur der Zeichnung Bezug genommen, die in schematischer Darstellung ein Ausführungsbeispiel der erfindungsgemäßen Anordnung zeigt.

Eine elektrische Anlage, z. B. eine Umspannstation, weist mehrere ölgefüllte elektrische Einrichtungen 1 bis 6, hier Transformatoren 1, 2, 3 und 5 sowie Drosseln 4 und 6, auf. Jeder der elektrischen Einrichtungen 1 bis 6 ist jeweils eine Einrichtung 7 bis 12 zur Extraktion der in dem Öl gelösten Gase zugeordnet, die jeweils ein gegenüber dem Öl durch eine semipermeable Membran abgetrenntes Gasaufnahmevolumen 13 bis 18 aufweist. Bei dem gezeigten Ausführungsbeispiel werden die Gasaufnahmevolumina 13 bis 18 jeweils von dem Inneren eines Schlauchs gebildet, dessen aus Polytetrafluoräthylen oder einem anderen geeigneten Material bestehende Schlauchwand die Membran bildet und der von dem Öl umspült wird. Beispiele hierfür sind aus der US-A-4 058 373 und US-A-4 112 737 bekannt.

Die unterschiedlichen Gasaufnahmevolumina 13 bis 18 sind jeweils über steuerbare Umschaltventile 19 bis 24 an einer an allen Einrichtungen 1 bis 6 vorbeiführenden Leitung 25 angeschlossen, die an einem Ende über ein Drosselventil 26 zur Dämpfung von Druckstößen mit einer Trägergasquelle 27 und an dem anderen Ende mit einer Gasanalyseeinrichtung 28 verbunden ist. Die Leitung 25 besteht bei dem gezeigten Ausführungsbeispiel aus einem polyimid-beschichteten Quarzrohr mit einem Innendurchmesser von 0,25 bis 0,5 mm, wie es auch für gaschromatographische Trennsäulen, dabei aber mit unbelegter Innenoberfläche, verwendet wird.

Die Umschaltventile 19 bis 24 sind einzeln durch eine Steuereinrichtung 29 schaltbar und in der Weise als Mehrwegeventile ausgebildet, daß in einer ersten, hier für das Umschaltventil 21 gezeigten Schaltstellung das zugehörige Gasaufnahmevolumen 15 in den Verlauf der Leitung 25 geschaltet ist und von dem Trägergas aus der Trägergasquelle 27 durchströmt wird. In einer zweiten, hier für die Umschaltventile 19, 20, 22, 23 und 24 gezeigten Schaltstellung ist das zugehörige Gasaufnahmevolumen 13, 14, 16, 17 und 18 jeweils in Reihe mit einem schaltbaren Ventil 30, hier einem Magnetventil, zwischen einem Gasabschluß 31 und der Atmosphäre geschaltet. Nach dem Ausspülen der Gasaufnahmevolumina 13 bis 18 mit dem Trägergas werden alle Gasaufnahmevolumina 13 bis 18 durch kurzzeitiges Öffnen der schaltbaren Ventile 30 auf gleichen Druck, hier den Atmosphärendruck, gebracht.

Die Gasanalyseeinrichtung 28, die hier z. B. aus zwei Trennsäulen 33 und nachgeordneten Gasdetektoren 34 besteht, ist über ein ebenfalls durch die Steuereinrichtung 29 steuerbares Dosierventil 35 in der Weise der Leitung 25 und an der Trägergasquelle 27 angeschlossen, daß in einer ersten, hier durch ausgezogene Linien dargestellten Ventilstellung die Gasanalyseeinrichtung 28 mit der Trägergasquelle 27 und die Leitung 25 mit einem Dosiervolumen 36 verbunden ist. Dabei ist das Dosiervolumen 36 zwischen die Leitung 25 und einen weiteren Gasdetektor 37 mit vorgeordnetem Magnetventil 38 und Drosselventil 39 geschaltet. Das Ausgangssignal des weiteren Gasdetektors 37 wird der Steuereinrichtung 29 zugeführt. In einer zweiten, hier gestrichelt dargestellten Ventilstellung ist das Dosiervolumen 36 zwischen die Trägergasquelle 27 und die Gasanalyseeinrichtung 28 geschaltet.

Die Steuereinrichtung 29 stellt an einem Ausgang 40 eine Umschaltinformation in Abhängigkeit von der Ansteuerung der Umschaltventile 19 bis 24 bereit, die zusammen mit den von der Gasanalyseeinrichtung 28 gelieferten Analyseergebnissen einer Einrichtung 41 zur Weiterverarbeitung dieser Informationen zugeführt wird. Dabei werden die Analyseergebnisse aufgrund der Umschaltinformation jeweils denjenigen der zu überwachenden Einrichtungen 1 bis 6 zugeordnet, von denen die analysierten Gase stammen. Die Weiterverarbeitung der Informationen in der Einrichtung 41 beinhaltet u. a. die Aufzeichnung der Anayseergebnisse und ihre Überwachung beispielsweise auf Grenzwertüberschreitungen.

Im folgenden wird die Funktion der dargestellten Anordnung beschrieben. Bei jeder der zu überwachenden ölgefüllten Einrichtungen 1 bis 6 diffundieren die in dem Öl gelösten Gase bei Normaldruck durch die semipermeable Membran in das jeweilige Gasaufnahmevolumen 13 bis 18 und werden dort angereichert. Der Grad der Anreicherung ist u. a. von der Löslichkeit der Gase, der Temperatur und den Partialdruckverhältnissen abhängig. Betriebsstörungen der elektrischen Einrichtungen 1 bis 6, wie z. B. deren Überhitzung und elektrische Entladungen, führen zu signifikanten Änderungen der Gasanteile in den Gasaufnahmevolumina 13 bis 18.

Bei den gezeigten Schaltstellungen der Umschaltventile 19 bis 24 werden die in dem der Einrichtung 3 zugeordneten Gasaufnahmevolumen 15 angereicherten Gase von dem Trägergas aus der Trägergasquelle 27 wie ein Pfropf durch die Leitung 25 an den Gasaufnahmevolumina 14 und 13 der Einrichtungen 2 und 1 vorbei in Richtung zu dem Dosierventil 35 geschoben. Dabei ist das Magnetventil 38 geöffnet. Aufgrund der mit durchgezogenen Linien dargestellten Ventilstellung des Dosierventils 35 gelangen die zu analysierenden Gase in das Dosiervolumen 36, wobei sich etwa die Mitte des Gaspfropfes in dem Dosiervolumen 36 befindet, wenn der Anfang des Gaspfropfes den Gasdetektor 37 erreicht. Inzwischen werden die Trennsäulen 33 der Gasanalyseeinrichtung 28 durch das Trägergas gereinigt.

Sobald der Gasdetektor 37 die Ankunft des Gaspfropfes detektiert, wird über die Steuereinrichtung 29 das Magnetventil 38 gesperrt, so daß der Gaspfropf nicht weiter transportiert wird. Jetzt wird das Dosierventil 35 durch die Steuereinrichtung 29 in die gestrichelt dargestellte Ventilstellung gesteuert, so daß das Dosiervolumen 36 zwischen die Trägergasquelle 27 und die Gasanalyseeinrichtung 28 geschaltet wird und die in dem Dosiervolumen 36 enthaltene definierte Menge der zu analysierenden Gase von dem Trägergas in die Gasanalyseeinrichtung 28 geschoben und dort analysiert wird.

Wenn der Gaspfropf aus dem Gasaufnahmevolumen 15 heraustransportiert worden ist, was hier z. B. durch die Ankunft des Gaspfropfes an dem Gasdetektor 37 detektiert wird, wird das Umschaltventil 21 durch die Steuereinrichtung 29 in seine zweite Schaltstellung gesteuert, die den für die Umschaltventile 19, 20, 22, 23 und 24 gezeigten Schaltstellungen entspricht. Durch Öffnen des entsprechenden Magnetventils wird daraufhin das Gasaufnahmevolumen 15 mit dem Spülgas durchspült und so für eine neue Gasanreicherung vorbereitet.

Auf die oben beschriebene Weise werden nacheinander alle den einzelnen zu überwachenden Einrichtungen 1 bis 6 zugeordneten Gasaufnahmevolumina 13 bis 18 in den Verlauf der Leitung 25 geschaltet und die angereicherten Gase der Gasanalyseeinrichtung 28 zugeführt.

## Patentansprüche

1. Anordnung zur Überwachung mehrerer mit Öl gefüllter elektrischer Einrichtungen (1 bis 6), in der jeder Einrichtung (1 bis 6) jeweils ein durch eine semipermeable Membran gegenüber dem Öl abgetrenntes Gasaufnahmevolumen (13 bis 18) zur Anreicherung von in dem Öl gelösten Gasen zugeordnet ist und jedes Gasaufnahmevolumen (13 bis 18) über ein ihm jeweils zugeordnetes Umschaltventil (19 bis 24) an einer Leitung (25) anschließbar ist, die an einem Ende mit einer Trägergasquelle (27) und an dem anderen Ende mit einer Gasanalyseeinrichtung (28) verbunden ist, wobei das Umschaltventil (19 bis 24) das Trägergas in einer ersten Schaltstellung durch das Gasaufnahmevolumen (13 bis 18) hindurch und in einer zweiten Schaltstellung an dem Gasaufnahmevolumen (13 bis 18) vorbeileitet, **dadurch gekennzeichnet, daß** alle Gasaufnahmevolumina (13 bis 18) über ihre Umschaltventile (19 bis 24) an der ihnen gemeinsamen Leitung (25) angeschlossen sind, daß eine Steuereinrichtung (29) zum zyklischen Umschalten der Umschaltventile (19 bis 24) und zur Bereitstellung einer entsprechenden Umschaltinformation vorhanden ist, daß die Gasanalyseeinrichtung (28) über ein steuerbares Dosierventil (35) an der Leitung (25) und an der Trägergasquelle (27) angeschlossen ist, wobei das Dosierventil (35) in einer ersten Ventilstellung die Leitung (25) mit einem Dosiervolumen (36) und die Gasanalyseeinrichtung (28) mit der Trägergasquelle (27) verbindet und in einer zweiten Ventilstellung das Dosiervolumen (36) zwischen die Trägergasquelle (27) und die Gasanalyseeinrichtung (28) schaltet, daß an dem Dosierventil (35) ein Gasdetektor (37) in der Weise angeschlossen ist, daß in der ersten Ventilstellung des Dosierventils (35) das Dosiervolumen (36) zwischen die Leitung (25) und den Gasdetektor (37) geschaltet ist und daß eine Einrichtung (29) zur Steuerung des Dosierventils (35) in Abhängigkeit von dem Ausgangssignal des Gasdetektors (37) vorhanden ist.

2. Anordnung nach Anspruch 1, **dadurch gekennzeichnet, daß** an den Umschaltventilen (z. B. 19) jeweils eine Spülgasquelle (30) in der Weise angeschlossen ist, daß in der zweiten Schaltstellung jedes Umschaltventils (19) das Gasaufnahmevolumen (13) jeweils in Reihe mit dem schaltbaren Ventil (30) zwischen einem Gasabschluß (31) und einer Referenzdruckquelle, insbesondere der Atmosphäre, geschaltet ist.

## Claims

1. An arrangement for monitoring a plurality of oil-filled electrical devices (1 to 6), in which a gas receiving volume (13 to 18) separated from the oil by a semi-permeable membrane is associated with each device (1 to 6) for the accumulation of gases dissolved in the oil, and each gas receiving volume (13 to 18) can be connected via a switching valve (19 to 24) associated with the respective gas receiving volume to a line (25), which is connected at one end to a carrier gas source (27) and at its other end to a gas analysing device (28), the switching valve (19 to 24) conveying the carrier gas in a first switching position through the gas receiving volume (13 to 18) and in a second switching position past the gas receiving volume (13 to 18), **characterised in that** all gas receiving volumes (13 to 18) are connected via their switching valves (19 to 24) to their common line (25), a control device (29) is provided for the cyclical switching of the switching valves (19 to 24) and for supplying corresponding switching data, the gas analysing device (28) is connected via a controllable metering valve (35) to the line (25) and to the carrier gas source (27), the metering valve (35) in a first valve position connecting the line (25) to a metering volume (36) and connecting the gas analysing device (28) to the carrier gas source (27) and in a second valve position connecting the metering volume (36) between the carrier gas source (27) and the gas analysing device (28), a gas detector (37) is connected to the metering valve (35) in such a manner that, in a first valve position of the metering valve (35), the metering volume (36) is connected between the line (25) and the gas detector (37), and a device (29) for controlling the metering valve (35) as a function of the output signal of the gas detector (37) is provided.

2. An arrangement according to claim 1, **characterised in that** a rinsing gas source (30) is connected to each of the switching valves (e.g. 19) in such a manner that, in the second switching position of each switching valve (19), the gas receiving volume (13) is connected in series in each case with the switchable valve (30) between a gas seal (31) and a reference pressure source, more particularly the atmosphere.

## Revendications

1. Système de surveillance de plusieurs dispositifs (1 à 6) électriques emplis d'huile, dans lequel il est associé à chaque dispositif (1 à 6) respectivement un volume (13 à 18) de réception de gaz séparé de l'huile par une membrane semi-perméable et destiné à s'enrichir en des gaz dissous dans l'huile et chaque volume (13 à 18) de réception des gaz peut être raccordé à un conduit (25) par une vanne (19 à 24) d'inversion qui lui et associée respectivement, qui communique par une extrémité avec une source (27) de gaz porteur et à l'autre extrémité avec un dispositif (28) d'analyse des gaz, la vanne (19 à 24) d'inversion faisant passer le gaz porteur dans une première position dans le volume (13 à 18) de réception de gaz et le faisant passer à côté du volume (13 à 18) de réception de gaz dans une deuxième position, **caractérisé en ce que** tous les volumes (13 à 18) de réception de gaz sont raccordés par leur vanne (19 à 24) d'inversion au conduit (25) qui leur est commun et **en ce qu'**il est prévu un dispositif (29) de commande pour l'inversion cyclique des vannes (19 à 24) d'inversion et pour la mise à disposition d'une information d'inversion correspondante, **en ce que** le dispositif (28) d'analyse des gaz est raccordé par une vanne (35) de dosage, qui peut être commandée, au conduit (25) et à la source (27) de gaz porteur, la vanne (35) de dosage mettant, dans une première position, le conduit (25) en communication avec un volume (36) de dosage et le dispositif (28) d'analyse des gaz avec la source (27) de gaz porteur et dans une deuxième position, mettant en circuit le volume (36) de dosage entre la source (27) de gaz porteur et le dispositif (28) d'analyse des gaz, **en ce qu'**il est raccordé à la vanne (35) de dosage un détecteur (37) de gaz de façon que dans la première position de la vanne (35) de dosage le volume (36) de dosage entre le conduit (25) et le détecteur (37) de gaz soit mis en circuit et **en ce qu'**il est prévu un dispositif (29) de commande de la vanne (35) de dosage en fonction du signal de sortie du détecteur (37) de gaz.

2. Système suivant la revendication 1, **caractérisé en ce qu'**il est raccordé aux vannes (par exemple 19) d'inversion respectivement une source (30) de gaz de rinçage de façon qu'en la deuxième position de chaque vanne (19) d'inversion, le volume (13) de réception des gaz soit respectivement en série avec la vanne (30) qui peut être fermée entre un arrêt (31) de gaz et une source de pression de référence, notamment l'atmosphère.
